# FASCICULE DE BREVET EUROPEEN

(11) **EP 3 701 989 B1**
(45) Date de publication et mention de la délivrance du brevet: **12.07.2023**
(21) Numéro de dépôt: 20158630.2
(22) Date de dépôt: 20.02.2020
(51) Int. Cl.: A61M 16/00, A61M 16/20, A61M 16/10

(54) **SYSTÈME D'ADMINISTRATION D'UN MÉLANGE GAZEUX POUR LUTTER CONTRE UNE DOULEUR CHRONIQUE**
SYSTEM ZUR VERABREICHUNG EINES GASGEMISCHES FÜR DIE BEHANDLUNG VON CHRONISCHEN SCHMERZEN
SYSTEM FOR ADMINISTERING A GASEOUS MIXTURE FOR THE TREATMENT OF CHRONIC PAIN

(30) Priorité: 27.02.2019 FR 1902001
(43) Date de publication de la demande: 02.09.2020
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: THOUVIER, Stéphane, 78354 Les Loges en Josas (FR); LECOURT, Laurent, 94250 Gentilly (FR)
(74) Mandataire: Air Liquide

(56) Documents cités:
- WO-A1-2016/005710
- FR-A1- 2 850 874
- FR-B1- 2 850 874
- US-A1- 2017 224 234

## Description

L'invention porte sur un système de délivrance d'un mélange gazeux binaire N₂O/O₂ constitué de protoxyde d'azote (N₂O) et d'oxygène (O₂), en particulier un mélange équimolaire ou MEOPA constitué de 50% de protoxyde d'azote et de 50% d'oxygène (% molaire).

Le mélange gazeux équimolaire (50%/50% molaire) de protoxyde d'azote et d'oxygène, couramment appelé MEOPA, a montré une efficacité dans le traitement des douleurs aigües ou chroniques de différentes étiologies, lorsqu'il est administré à un individu, i.e. un patient, par inhalation au moyen d'une interface respiratoire, tel un masque respiratoire, des canules nasales ou analogue.

La durée d'administration du MEOPA chez des patients souffrant de douleurs chroniques, appelés « patients chroniques », de différentes étiologies est plus longue que celle classiquement observée lors de traitement de douleurs aigues, appelés « patients aigus », et les administrations sont répétées dans le temps. Cette terminologie « patients chroniques » et « patients aigus » est utilisée ci-après dans un but de simplification.

Les systèmes et autres appareils de fourniture de MEOPA aux patients chroniques doivent donc être différents de ceux couramment utilisés pour traiter les patients aigus, notamment car les patients chroniques ne sont pas forcément hospitalisés, c'est-à-dire qu'ils sont souvent traités dans des structures de soins en dehors de l'hôpital ou à leur domicile avec des surveillances médicales beaucoup plus légères, voire absentes.

De fait de cet usage hors hôpital, les systèmes de délivrance de MEOPA doivent être conçus pour empêcher ou limiter tout mésusage du MEOPA par les patients chroniques, notamment tout risque d'erreur dans la posologie délivrée ( dose durée maximale de la cure et nombre de session ) à ces patients, et par ailleurs pour empêcher tout usage détourné ou non-conforme du MEOPA, notamment par des personnes non malades qui seraient tentées de l'inhaler pour tirer parti de ses propriétés euphorisantes à des fins récréatives.

En outre, les systèmes de délivrance de MEOPA doivent être conçus pour délivrer du MEOPA conforme aux spécifications des dossiers d'autorisation de mise sur le marché du médicament (AMM), en termes de qualité et de conformité pharmaceutique des teneurs de chaque principe actif (N₂O et O₂) et impuretés éventuelles, notamment en prenant en compte le risque de dé-mélange du MEOPA. Ce démélange pouvant se produire lorsque la bouteille est stockée à basse température (- 5°C), cette température de démélange pouvant varier en fonction de la pression dans la bouteille, cette limite de -5°C est donnée pour une pression de 170 bar. A cette température, le protoxyde d'azote gazeux va passer en phase liquide, le mélange gazeux ne sera plus homogène et le patient inhalera, en fonction du stade de ce démélange, un mélange plus riche en oxygène au début (i.e. pas d'effet antalgique) et ensuite, le patient recevra du protoxyde d'azote à un pourcentage > à 50% et donc susceptible de provoquer une sédation profonde ou une asphyxie si le mélange ne contient plus d'oxygène.

Cette problématique de démélange à basse température entraîne la mise en place de procédures de stockage très strictes et contraignantes à l'hôpital et de préparation du gaz avant administration, notamment un stockage et une gestion des bouteilles en position horizontale pendant 48 heures avant utilisation.

Ces procédures sont difficiles à mettre en oeuvre dans une structure hospitalière et sont impossibles à maîtriser dans une structure de soins en dehors de l'hôpital.

On connait par ailleurs US-A-2017/224234 qui enseigne un appareil de ventilation de patient utilisable en milieu hospitalier comprenant un mélangeur de gaz venant se raccorder aux prises murales fournissant l'oxygène et l'air, et des capteurs bioélectriques mesurant des signaux bioélectriques chez le patient, tel que l'activité du diaphragme, l'activité cardiaque (ECG), la saturation en oxygène (SpO₂). Il permet de réaliser des mélanges air/oxygène qui sont administrés ensuite au patient.

Au vu de cela, le problème est de proposer un système de délivrance d'un mélange gazeux binaire N₂O/O₂ constitué de protoxyde d'azote (N₂O) et d'oxygène (O₂), en particulier de MEOPA, c'est-à-dire d'un mélange gazeux équimolaire 50%/50% (% molaire) de protoxyde d'azote et d'oxygène, qui soit adapté à une fourniture de ce mélange gazeux à des patients chroniques, lequel ne présente pas les inconvénients et autres problèmes susmentionnés, notamment ceux liés à une utilisation du MEOPA ou d'un autre mélange N₂O/O₂, à l'hôpital, ou dans des lieux de soins dans lesquels la surveillance médicale est moins présente, voir absente, en particulier au domicile des patients traités.

Le document de brevet FR2850874A1 divulgue un conteneur de gaz contenant un mélange équimolaire de N2O/O2.

La solution selon la présente invention concerne alors un système de délivrance d'un mélange gazeux N₂O/O₂ constitué de protoxyde d'azote (N₂O) et d'oxygène (O₂), lequel système est adapté à une utilisation par un patient souffrant de douleurs chroniques, qu'il soit traité dans une structure médicalisée ou dans une structure avec surveillance médicale plus légère, tel que médecin généraliste, centre de rééducation, kinésithérapeute, cabinet d'infirmiers..., ou absente, en particulier au domicile des patients, comprenant un récipient de gaz contenant un mélange N₂O/O₂ contenant une teneur en N₂O comprise entre 40 et 60% mol., et de l'oxygène pour le reste, relié fluidiquement à un dispositif de distribution de gaz, caractérisé en ce que le dispositif de distribution de gaz comprend :
- un passage interne de gaz pour acheminer le gaz au sein du dispositif de distribution de gaz, ledit passage interne de gaz comprenant une entrée de gaz et se ramifie en une première et une seconde branche munie chacune d'une sortie de gaz,
- des moyens d'analyse pour mesurer une teneur en oxygène du mélange gazeux N₂O/O₂ et fournir la teneur mesurée à des moyens de pilotage,
- des moyens de pilotage configurés pour comparer la teneur en oxygène mesurée (TOxₘₑₛ) fournie par les moyens d'analyse à une teneur en oxygène souhaitée (TOx_{fix}),
- des moyens de contrôle de la fourniture de gaz conçus pour empêcher ou pour interrompre la distribution du gaz par le dispositif de distribution de gaz lorsque les moyens de pilotage déterminent une teneur en oxygène mesurée (TOxₘₑₛ) différente à la teneur en oxygène souhaitée (TOx_{fix}), et
- une interface homme-machine, aussi appelée IHM, configurée pour permettre à un utilisateur au moins de sélectionner un mode de délivrance du mélange gazeux en débit ou en pression, et
- des moyens de commutation pour fournir du gaz à la première branche ou, alternativement, à la seconde branche du passage interne de gaz en fonction du mode de délivrance sélectionné par l'utilisateur sur l'interface homme-machine.

Dans le cadre de l'invention, les teneurs en O₂ et en N₂O sont exprimées en pourcentages molaires ou « % mol. » ; toutefois, on pourrait aussi les exprimer en pourcentages volumiques ou « % vol. ».

Selon le mode de réalisation considéré, l'invention peut comprendre l'une ou plusieurs des caractéristiques suivantes :
- le récipient de gaz contient un mélange gazeux N₂O/O₂ constitué de 45 à 55% mol. de protoxyde d'azote et de l'oxygène pour le reste.
- le récipient de gaz contient un mélange gazeux N₂O/O₂ constitué de 48 à 52% mol. de protoxyde d'azote et de l'oxygène pour le reste.
- le récipient de gaz contient un mélange gazeux N₂O/O₂ constitué de 49 à 51% mol. de protoxyde d'azote et de l'oxygène pour le reste.
- le récipient de gaz contient un mélange gazeux équimolaire N₂O/O₂ ou MEOPA, c'est-à-dire constitué de 50 %mol. de N₂O et d'oxygène (O₂) pour le reste, et éventuellement des impuretés inévitables (i.e., +/- 1 %mol. au maximum).
- la teneur en oxygène souhaitée (TOx_{fix}) est préfixée en fonction du mélange gazeux N₂O/O₂ mis en oeuvre, c'est-à-dire provenant du récipient de gaz.
- le récipient de gaz contient le mélange gazeux à une pression inférieure ou égale à 350 bar abs, de préférence d'au moins 10 bar abs, de préférence encore d'au moins 50 bar abs.
- le récipient de gaz est une bouteille de gaz.
- le récipient de gaz est une bouteille de gaz en acier ou en un alliage d'aluminium, par exemple un alliage de type 6061, 7032 ou 7060.
- le récipient de gaz est une bouteille de gaz en matériaux composites.
- le récipient de gaz est une bouteille de gaz de contenance comprise entre 1 et 20 L (volume en eau).
- le récipient de gaz est une cartouche monodose, c'est-à-dire à usage unique, de volume inférieur à 5 L, de préférence inférieur ou égal à 2 L, par exemple de 1L ou moins.
- le récipient de gaz est une bouteille de gaz équipée d'une vanne de distribution soit de type robinet simple, soit de type robinet à détendeur intégré (RDI) muni d'un raccord de sortie de gaz à basse pression et d'un dispositif indicateur de pression permettant de fournir, notamment d'afficher, la pression haute régnant dans la bouteille, c'est-à-dire dans le volume interne délimité par le corps de la bouteille.
- le récipient de gaz est équipé de préférence d'un RDI.
- le récipient de gaz est équipé d'un capotage de protection, encore appelé « chapeau de bouteille» servant à protéger la vanne de distribution, i.e. le robinet.
- le RDI est muni uniquement d'un raccord de sortie de gaz à basse pression, appelée « sortie BP », délivrant le mélange N₂O/O₂ à une pression inférieure ou égale à 5 bar abs.
- le récipient de gaz est relié fluidiquement au dispositif de distribution de gaz via le raccord de sortie de gaz à basse pression:
   ▪ soit directement, c'est-à-dire qu'elle se connecte elle-même au dispositif de distribution de gaz via un système de connexion adapté,
   ▪ soit indirectement, c'est-à-dire au moyen d'une canalisation de gaz flexible, c'est-à-dire un tuyau flexible apte à acheminer le gaz à basse pression délivré par le raccord de sortie de gaz à basse pression de la bouteille.
- avantageusement, le raccord de sortie de gaz à basse pression est muni d'un système de détrompage ne permettant pas le raccordement fluidique d'un dispositif non-autorisé et ce, de manière à empêcher les mésusages du gaz.
- les moyens d'analyse sont agencés de manière à mesurer la teneur en oxygène du mélange gazeux N₂O/O₂ au sein du passage interne de gaz.
- les moyens d'analyse comprennent un capteur et/ou un analyseur de gaz.
- le passage interne de gaz comprend un conduit ou analogue.
- les moyens de pilotage comprennent une carte électronique comprenant un (ou plusieurs) microprocesseur, en particulier un microcontrôleur.
- il comprend en outre des moyens d'alarme configurés pour déclencher une alarme sonore et/ou visuelle lorsque les moyens de pilotage déterminent une teneur en oxygène mesurée (TOxₘₑₛ) différente de la teneur en oxygène souhaitée (TOx_{fix}).
- ladite première branche est une branche dite « en pression » permettant d'y raccorder un système d'administration de type valve à la demande. La valve à la demande peut être soit intégrée dans le système, soit connectée à la sortie de la première branche (i.e. à basse pression). Cette première branche achemine du gaz à une pression égale ou équivalente à celle régnant dans passage interne de gaz, par exemple de l'ordre de 4,5 bar abs.
- la seconde branche est une branche dite « en débit », c'est-à-dire délivrance le gaz au débit souhaité et à une pression inférieure à celle de la première branche, appelée basse pression, par exemple de l'ordre de 1 bar abs.
- la seconde branche dite « en débit » comprend des moyens d'ajustage de débit, par exemple un ou des orifices calibrés ou une ou des électrovannes ou tout autre dispositif adapté permettant de fixer différents débits.
- les moyens de commutation comprennent une vanne 3 voies commandée.
- les moyens de commutation comprennent en particulier une électrovanne (EV) , telle une électrovanne à plusieurs voies.
- les moyens de commutation sont agencés sur le passage de gaz.
- les moyens de commutation sont pilotés par les moyens de pilotage.
- les moyens de commutation sont pilotés par les moyens de pilotage en réponse à une sélection par l'utilisateur d'un mode de délivrance de gaz (i.e. en pression ou en débit).
- l'IHM comprend des moyens d'entrée de données et/ou des moyens d'affichage d'informations.
- l'IHM est en outre configurée pour réaliser une ou d'autres fonctions, notamment une fixation du temps de délivrance du gaz (i.e. temps de la cure), une gestion des temps de pause....
- les moyens d'entrée de données comprennent des touches, des boutons, des curseurs, en particulier un clavier virtuel, i.e. digital.
- les moyens d'affichage d'informations comprennent un écran ou un afficheur, par exemple un écran tactile, notamment en couleurs.
- l'IHM est configurée (i.e. conçue) pour permettre à un utilisateur de fixer en outre une durée d'administration de gaz et une durée totale de session de soins (i.e. cure). Dans le cadre de l'invention, on appelle :
   ▪ durée d'administration de gaz : la durée (exprimée en minutes) pendant laquelle du mélange N₂O/O₂ est fourni au patient, lequel inhale le mélange N₂O/O₂.
   ▪ durée totale de session de soins : la durée (exprimée en minutes) pendant laquelle le patient exécute une session de soins laquelle inclut une période pendant laquelle le patient inhale le mélange N₂O/O₂ et une (ou plusieurs) période pendant laquelle le patient n'inhale pas le mélange N₂O/O₂, en particulier une période de récupération post-inhalation du gaz.
- par exemple, la durée d'administration de gaz peut être fixée entre 30 min et 1 h30, de préférence de l'ordre de 55 à 60 minutes lors d'un traitement de la douleur chronique.
- par exemple, la durée totale de session de soins est variable en fonction du nombre de pauses. Hors pauses, la durée de la session correspond à la durée d'administration de gaz à laquelle vient s'ajouter une période dite de récupération de l'ordre de 5 à 20 minutes.
- l'IHM est en outre configurée (i.e. conçue) pour permettre à un utilisateur de choisir un mode de délivrance de gaz du type en débit et de fixer un débit de délivrance souhaité, notamment en fonction du volume/minute du patient.
- les moyens d'alarme coopèrent avec l'IHM pour afficher sur l'écran de visualisation de l'IHM une alerte visuelle indiquant une teneur en oxygène mesurée (TOxₘₑₛ) inférieure à la teneur en oxygène souhaitée (TOx_{fix}).
- les moyens d'alarme sont configurés pour déclencher une alarme pour signaler une teneur en oxygène mesurée (TOxₘₑₛ) inférieure à la teneur en oxygène souhaitée (TOx_{fix}) résultant d'une erreur de source de pré-mélange (i.e. mauvaise bouteille de gaz), d'un problème de démélange du produit lié à un stockage ou passage à basse température.
- il comprend en outre des moyens de mémorisation configurés pour enregistrer et/ou mémoriser les paramètres (i.e. mesures, données...) de la (ou de plusieurs) séance de soins, notamment durée de la séance, le nombre de pauses, le débit et la durée de séance pour un même patient avec possibilité de pré-réglage pour la cure suivante...
- une interface respiratoire patient, par exemple un masque respiratoire, un embout buccal ou des canules nasales, est reliée fluidique aux sorties de gaz du dispositif, en particulier au moyen d'un tuyau flexible ou analogue permettant l'acheminement du mélange gazeux jusqu'au patient.
- il comprend par ailleurs des moyens d'alimentation électrique, telle une prise électrique secteur munie d'un cordon électrique, ou une (ou plusieurs) batterie ou analogue, notamment une batterie rechargeable.
- il comprend une coque ou carcasse externe dans laquelle sont agencés tout ou partie des éléments composant le dispositif de distribution de gaz.

L'invention va maintenant être mieux comprise grâce à la description détaillée suivante, faite à titre illustratif mais non limitatif, en référence à :
[Fig .1] qui représente un schéma de principe d'un mode de réalisation d'un système de fourniture de gaz selon la présente invention.

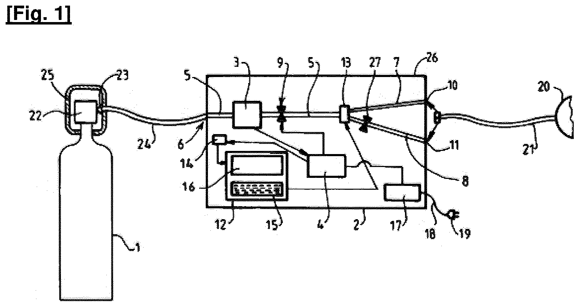

[Fig. 1] représente un schéma de principe selon un mode de réalisation d'un système de fourniture d'un mélange gazeux N₂O/O₂ selon la présente invention, à savoir ici un mélange équimolaire (50/50 %mol.) constitué de protoxyde d'azote (N₂O) et d'oxygène (O₂), c'est-à-dire de MEOPA.

Ce système de délivrance comprend un récipient de gaz 1 contenant le mélange N₂O/O₂, à savoir ici du MEOPA, qui est relié fluidiquement à un dispositif de distribution de gaz 2, ce dernier alimentant une interface respiratoire patient 20, tel un masque respiratoire par exemple, via un tuyau flexible 21 servant à acheminer le MEOPA depuis le dispositif de distribution de gaz 2 jusqu'à l'interface respiratoire patient 20.

Le récipient de gaz 1 est ici une bouteille de MEOPA (i.e. mélange 50% N₂O/50% O₂) équipée d'une vanne de distribution 22 dont le raccord de sortie 23 est relié fluidiquement au système de délivrance 2 de l'invention par un conduit flexible 24 à basse pression permettant d'acheminer le MEOPA sous une pression inférieure ou égale à 5 bar abs, fourni par le raccord de sortie 23 de la vanne de distribution 22 de la bouteille 1 de MEOPA. La bouteille 1 permet de stocker le MEOPA à haute pression, c'est-à-dire à une pression pouvant atteindre plusieurs centaines de bar abs, par exemple au moins 200, 300 ou 350 bar abs.

La vanne de distribution 22 est ici un robinet à détendeur intégré (RDI) permettant de réduire la pression du MEOPA jusqu'à la basse pression (< 5 bar abs). De préférence, la vanne de distribution 22 est protégée par un capotage de protection 25, aussi appelé chapeau de bouteille, en métal et/ou en polymère.

Selon la présente invention, le dispositif de distribution de gaz 2 comprend un boîtier 26 externe rigide traversé par un passage interne de gaz 5, tel un conduit ou analogue, servant à acheminer le gaz dans le dispositif de distribution de gaz 2 entre une entrée de gaz 6 et plusieurs sorties 10, 11.

En effet, le passage interne de gaz 5 comprenant une entrée de gaz 6 reliée à la bouteille 1, et se ramifie par ailleurs en une première 7 et une seconde branche 8 munie chacune d'une sortie de gaz 10, 11. La première branche 7 est une branche dite « en pression », comprenant une sortie de gaz « en pression » 10, alors que la seconde branche 8 est une branche dite « en débit », comprenant une sortie de gaz « en débit » 11.

L'utilisateur peut choisir d'envoyer le gaz vers l'une ou l'autre des branches 7, 8 en fonction du mode de délivrance souhaité et informé au niveau de l'IHM 12, comme expliqué ci-après.

Sont prévus par ailleurs des moyens d'analyse 3, par exemple une sonde ou un capteur d'oxygène pour mesurer la teneur en oxygène du mélange gazeux N₂O/O₂ au sein du passage interne de gaz 5 et fournir la teneur mesurée (TOxₘₑₛ) à des moyens de pilotage 4, telle une carte électronique à microcontrôleur, qui vont traiter ces mesures de teneur en O₂ (i.e. les signaux de mesure) et les comparer avec une teneur en oxygène souhaitée (TOx_{fix}).

Cette comparaison de teneurs en oxygène permet de s'assurer que le mélange gazeux est bien conforme, par exemple qu'il contient bien 50%mol. d'oxygène dans le cas du MEOPA, et manière à éviter tout risque pour le patient qui doit l'inhaler.

En cas de détection d'une teneur non-conforme, c'est-à-dire lorsque la teneur en oxygène mesurée (TOxₘₑₛ) est différente de la teneur en oxygène souhaitée (TOx_{fix}), des moyens d'alarme 14 déclenchent une alarme sonore et/ou visuelle afin d'alerter l'utilisateur qu'un problème de teneur non-conforme a été décelée. Par exemple, une alarme visuelle peut être affichée sur l'afficheur 16 d'une IHM 12.

De manière (quasi) simultanée, des moyens de contrôle 9 de la fourniture de gaz, telle une électrovanne, agencés sur le passage interne de gaz 5, sont commandés par les moyens de pilotage pour empêcher ou interrompre la distribution du gaz par le dispositif de distribution de gaz 2.

Par ailleurs, le dispositif de distribution de gaz 2 comprend aussi une interface homme-machine ou IHM 12 conçue et/ou configurée pour permettre à un utilisateur notamment de fixer la teneur en oxygène souhaitée (TOx_{fix}), par exemple 50%mol. d'O₂. L'IHM 12 comprend par exemple un afficheur 16 et un clavier 15 d'entrée de données, d'ordres, de validation de consignes ou analogue.

L'IHM 12 permet aussi de sélectionner un mode de délivrance du mélange gazeux, à savoir un mode en débit ou un mode en pression, de sorte d'envoyer le gaz vers l'une ou l'autre des première et seconde branches 7, 8 du circuit de gaz 5. Ceci se fait grâce à des moyens de commutation 13 agencés sur le passage interne de gaz 5, en particulier à l'endroit où il se ramifie en lesdites première et seconde branches 7, 8. Ces moyens de commutation 13, tel un sélecteur, permettent de fournir le MEOPA à la première branche 7 ou, alternativement, à la seconde branche 8 du passage interne de gaz 5 en fonction du mode de délivrance sélectionné par l'utilisateur sur l'IHM 12.

Afin de permettre la conversion « en débit » du gaz sous pression, on prévoit sur la seconde branche 8, un dispositif de débitmétrie, c'est-à-dire des moyens d'ajustage de débit 27, par exemple un ou des orifices calibrés ou une ou des électrovannes ou tout autre dispositif adapté permettant de fixer différents débits.

Le dispositif de débitmétrie 27 est agencé sur la seconde branche 8, en aval des moyens de commutation 13.

D'une façon générale, l'IHM 12 permet à un utilisateur, par exemple un personnel soignant, voire le patient lui-même, de saisir ou sélectionner différents choix ou paramètres de fonctionnement et/ou liés au traitement à réaliser, en particulier la durée totale des soins, le débit de gaz à administrer, le mode de délivrance (débit ou pression)... Ces choix ou sélections ne peuvent avantageusement se faire qu'après avoir saisi un identifiant et/ou code de saisie, qui permet de débloquer le système.

L'IHM 12 comprend préférentiellement un écran tactile, avantageusement à affichage en couleurs.

A titre d'exemple, pour une administration de gaz en mode de délivrance en débit, l'utilisateur entre via l'IHM 12, un débit souhaité de MEOPA. Ce débit pourra ensuite être régulé de manière manuelle ou automatique.

Pour ce faire, l'IHM 12 peut proposer plusieurs choix de débits préenregistrés en fonction du type de patient à traiter, par exemple:
- choix « 1 » correspondant à un débit préfixé de 4 L/min de MEOPA,
- choix « 2 » correspondant à un débit préfixé de 7 L/min de MEOPA,
- choix « 3 » correspondant à un débit préfixé de 10 L/min de MEOPA et
- choix « 4 » correspondant à un débit préfixé de 12 L/min de MEOPA.

De manière alternative, l'utilisateur ou le personnel soignant peut aussi saisir le débit de gaz en sortie du dispositif qui est le plus adapté aux besoins du patient, typiquement un débit adapté au volume/minute du patient en prenant en compte l'interface respiratoire 20 utilisée pour assurer la délivrance du gaz au patient, par exemple un masque facial. Par exemple, le débit de gaz de sortie, à savoir un mélange N₂O/O₂/N₂, peut être compris entre 6 et 20 L/min, typiquement inférieur ou égal à 15 L/min.

Avantageusement, pour éviter tous les risques associés à un mésusage ou usage récréatif du MEOPA, le dispositif peut aussi être équipé d'un système de verrouillage permettant de limiter les utilisateurs autorisés ainsi que le nombre d'administration par patient conformément aux prescriptions médicales.

Par ailleurs, le dispositif peut aussi comprendre des moyens de mémorisation (i.e. carte mémoire ou autre) permettant d'enregistrer et retrouver les informations sur le dernier mois d'utilisation ou une autre période de temps donnée.

Par ailleurs, des moyens d'alimentation électrique 17-19, telle une prise électrique 19 et un cordon de raccordement 18 au secteur (1 10/220V) et/ou une (ou plusieurs) batterie 17, alimentent en courant électrique, les éléments du système ayant besoin d'énergie électrique pour fonctionner, en particulier les moyens de pilotage 4, telle une carte électronique à microprocesseur.

Tout ou partie des composants du système, sont agencés au sein d'un boîtier 26 formant carcasse externe.

Il est à noter que le MEOPA peut être humidifié et/ou chauffé avant sa fourniture au patient, grâce à par exemple un humidificateur de gaz (non montré) venant se raccorder en sortie du dispositif de distribution de gaz 2 selon l'invention.

La durée totale d'administration de gaz doit être conforme à la prescription médicale. Par exemple, dans le cas d'une douleur chronique, il est recommandé d'opérer une administration de gaz pendant 1 heure environ.

Le temps d'inhalation est préférentiellement affiché sur l'écran 16 de l'IHM 12. L'arrêt de l'administration est opéré lorsque la durée prescrite est écoulée. Préférentiellement, on opère aussi une détermination et un enregistrement de l'observance du patient à son traitement. Bien entendu, d'autres durées peuvent être fixées en fonction du traitement à mettre en oeuvre.

La durée d'administration est limitée et le gaz n'est plus délivré après une période prédéterminée, conformément à la prescription médicale. Le système permet un suivi post administration, la récupération du patient et le retour à son état de vigilance de base qui sera validé au moyen d'informations transmises par le patient et validé par le personnel soignant.

On peut prévoir aussi d'autres moyens de sécurité, par exemple l'initiation du traitement et le démarrage de la fourniture du mélange gazeux au patient peut se faire uniquement après validation d'un code d'identification du patient et/ou du personnel soignant. De même, on peut prévoir un système de contrôle de dose pour limiter la dose maximale quotidienne délivrée à un même patient et ce, notamment pour éviter les mésusages et/ou un surdosage éventuel.

Les informations sont mémorisées et/ou transmises par exemple au médecin en charge du patient considéré à des fins de validation de l'observance et des doses administrées (e.g., durée de la cure, débit administré, nombre de pauses durant la séance. On peut aussi suivre et enregistrer d'autres paramètres physiologiques, comme les teneurs en oxygène mesurées (SpO₂) ou la fréquence respiratoire qui pourrait permettre de prévenir des risques liés au produit sur certaine population

La délivrance du mélange gazeux de la manière la plus adaptée au patient soit par l'intermédiaire d'une valve à la demande, soit la délivrance du débit le mieux adapté au patient.

Le réglage de la durée exacte de la cure, ne permettant pas de surdosage, permet le respect de la prescription et de la bonne posologie. L'IHM 12 peut aussi permettre une mise en "stand-by" de l'administration du gaz en cas de pause du patient, tel que fatigue ou événement indésirable par exemple. Le réglage d'une durée de 15 à 30 minutes post-administration permettra de confirmer la reprise des fonctions cognitives du patient et de vérifier le retour à un état de conscience identique à celui avant la cure, i.e. les soins.

Des fonctions complémentaires de ce système permettent de suivre les cures et d'enregistrer des paramètres relatifs au patient, e.g. enregistrés et/ou transmis au médecin traitant pour vérifier l'adhérence au traitement ainsi que l'efficacité et la tolérance de la cure. Ces fonctions d'enregistrements et de transferts des informations relatives à la cure sont d'autant plus importantes que le patient se trouve dans un milieu peu ou pas médicalisé, par exemple à son domicile.

L'intérêt d'un tel dispositif est qu'il permet d'éviter le risque de surdosage et ses conséquences néfastes pour le patient (i.e. sédation profonde, asphyxie...) toute en facilitant gestion des bouteilles de gaz et logistique.

## Revendications

1. Système de délivrance d'un mélange gazeux N₂O/O₂ de protoxyde d'azote (N₂O) et d'oxygène (O₂) comprenant un récipient de gaz (1) contenant ledit mélange N₂O/O₂ contenant une teneur en N₂O comprise entre 40 et 60% mol., et de l'oxygène pour le reste, relié fluidiquement à un dispositif de distribution de gaz (2), dans lequel le dispositif de distribution de gaz (2) comprend :
- un passage interne de gaz (5) pour acheminer le gaz au sein du dispositif de distribution de gaz (2), ledit passage interne de gaz (5) comprenant une entrée de gaz (6) et se ramifie en une première (7) et une seconde branche (8) munie chacune d'une sortie de gaz (10, 11),
- des moyens d'analyse (3) pour mesurer une teneur en oxygène du mélange gazeux N₂O/O₂ et fournir la teneur mesurée à des moyens de pilotage (4),
- des moyens de pilotage (4) configurés pour comparer la teneur en oxygène mesurée (TOxₘₑₛ) fournie par les moyens d'analyse (3) à une teneur en oxygène souhaitée (TOx_{fix}),
- des moyens de contrôle (9) de la fourniture de gaz conçus pour empêcher ou pour interrompre la distribution du gaz par le dispositif de distribution de gaz (2) lorsque les moyens de pilotage (4) déterminent une teneur en oxygène mesurée (TOxₘₑₛ) différente de la teneur en oxygène souhaitée (TOx_{fix}),
- une interface homme-machine (12) configurée pour permettre à un utilisateur au moins de sélectionner un mode de délivrance du mélange gazeux en débit ou en pression, et
- des moyens de commutation (13) pour fournir du gaz à la première branche (7) ou, alternativement, à la seconde branche (8) du passage interne de gaz (5) en fonction du mode de délivrance sélectionné par l'utilisateur sur l'interface homme-machine (12).

2. Système selon la revendication précédente, **caractérisé en ce que** le récipient de gaz (1) contient un mélange gazeux N₂O/O₂ contenant entre 45 et 55% mol. de protoxyde d'azote et de l'oxygène pour le reste.

3. Système selon l'une des revendications précédentes, **caractérisé en ce que** le récipient de gaz (1) contient un mélange gazeux équimolaire N₂O/O₂.

4. Système selon l'une des revendications précédentes, **caractérisé en ce que** les moyens d'analyse (3) sont agencés de manière à mesurer la teneur en oxygène du mélange gazeux N₂O/O₂ au sein du passage interne de gaz (5).

5. Système selon l'une des revendications précédentes, **caractérisé en ce qu'**il comprend en outre des moyens d'alarme (14) configurés pour déclencher une alarme sonore et/ou visuelle lorsque les moyens de pilotage (4) déterminent une teneur en oxygène mesurée (TOxₘₑₛ) différente de la teneur en oxygène souhaitée (TOx_{fix}).

6. Système selon l'une des revendications précédentes, **caractérisé en ce que** ladite première branche (7) est une branche en pression et ladite seconde branche (8) est une branche en débit.

7. Système selon l'une des revendications précédentes, **caractérisé en ce que** les moyens de commutation (13) comprennent une électrovanne.

8. Système selon l'une des revendications précédentes, **caractérisé en ce que** l'interface homme-machine (12) comprend des moyens d'entrée de données (15) et/ou des moyens d'affichage d'informations (16).

9. Système selon l'une des revendications précédentes, **caractérisé en ce que** l'interface homme-machine (12) est configurée pour permettre à un utilisateur de fixer une durée d'administration de gaz et une durée totale de session de soins.

10. Système selon l'une des revendications précédentes, **caractérisé en ce que** la seconde branche (8) comprend des moyens d'ajustage de débit (27).

## Patentansprüche

1. System zur Abgabe eines Gasgemisches N₂O/O₂ aus Distickstoffmonoxid (N₂O) und Sauerstoff (O₂), umfassend einen Gasbehälter (1), der das Gemisch N₂O/O₂ enthält, das einen N₂O-Gehalt zwischen 40 und 60 Mol-% und für den Rest Sauerstoff enthält, und der fluidisch mit einer Gasverteilungsvorrichtung (2) verbunden ist, wobei die Gasverteilungsvorrichtung (2) umfasst:
- einen internen Gasdurchlass (5) zum Befördern des Gases in der Gasverteilungsvorrichtung (2), wobei der interne Gasdurchlass (5) einen Gaseinlass (6) umfasst und sich in einen ersten (7) und einen zweiten Zweig (8) verzweigt, die jeweils mit einem Gasauslass (10, 11) versehen sind,
- Analysemittel (3) zum Messen eines Sauerstoffgehalts des Gasgemisches N₂O/O₂ und Ausgeben des gemessenen Gehalts an Steuerungsmittel (4),
- Steuerungsmittel (4), die dazu ausgestaltet sind, den gemessenen Sauerstoffgehalt (TOxₘₑₛ), der von den Analysemitteln (3) ausgegeben wurde, mit einem gewünschten Sauerstoffgehalt (TOx_{fix}) zu vergleichen,
- Kontrollmittel (9) zum Kontrollieren der Gasausgabe, die dazu ausgestaltet sind, die Verteilung des Gases durch die Gasverteilungsvorrichtung (2) zu verhindern oder zu unterbrechen, wenn die Ansteuerungsmittel (4) einen gemessenen Sauerstoffgehalt (TOxₘₑₛ) feststellen, der von dem gewünschten Sauerstoffgehalt (TOx_{fix}) abweicht,
- eine Mensch-Maschine-Schnittstelle (12), die dazu ausgestaltet ist, einem Benutzer zu ermöglichen, mindestens eine volumenstrombezogene oder druckbezogene Abgabeart des Gasgemisches zu wählen, und
- Umschaltmittel (13), um Gas in Abhängigkeit von der Abgabeart, die von dem Benutzer an der Mensch-Maschine-Schnittstelle (12) gewählt wurde, an den ersten Zweig (7) oder alternativ an den zweiten Zweig (8) des internen Gasdurchlasses (5) auszugeben.

2. System nach dem vorhergehenden Anspruch, **dadurch gekennzeichnet, dass** der Gasbehälter (1) ein Gasgemisch N₂O/O₂ enthält, das zwischen 45 und 55 Mol-% Distickstoffmonoxid und für den Rest Sauerstoff enthält.

3. System nach einem der vorhergehenden Ansprüche, **dadurch gekennzeichnet, dass** der Gasbehälter (1) ein äquimolares Gasgemisch N₂O/O₂ enthält.

4. System nach einem der vorhergehenden Ansprüche, **dadurch gekennzeichnet, dass** die Analysemittel (3) so gestaltet sind, dass sie den Sauerstoffgehalt des Gasgemisches N₂O/O₂ in dem internen Gasdurchlass (5) messen.

5. System nach einem der vorhergehenden Ansprüche, **dadurch gekennzeichnet, dass** es ferner Alarmmittel (14) umfasst, die dazu ausgestaltet sind, einen akustischen und/oder optischen Alarm auszulösen, wenn die Ansteuerungsmittel (4) einen gemessenen Sauerstoffgehalt (TOxₘₑₛ) feststellen, der von dem gewünschten Sauerstoffgehalt (TOx_{fix}) abweicht.

6. System nach einem der vorhergehenden Ansprüche, **dadurch gekennzeichnet, dass** der erste Zweig (7) ein druckbezogener Zweig ist und dass der zweite Zweig (8) ein volumenstrombezogener Zweig ist.

7. System nach einem der vorhergehenden Ansprüche, **dadurch gekennzeichnet, dass** die Umschaltmittel (13) ein Magnetventil umfassen.

8. System nach einem der vorhergehenden Ansprüche, **dadurch gekennzeichnet, dass** die Mensch-Maschine-Schnittstelle (12) Dateneingabemittel (15) und/oder Informationsanzeigemittel (16) umfasst.

9. System nach einem der vorhergehenden Ansprüche, **dadurch gekennzeichnet, dass** die Mensch-Maschine-Schnittstelle (12) dazu ausgestaltet ist, einem Benutzer zu ermöglichen, eine Gasverabreichungsdauer und eine Behandlungssitzungsgesamtdauer festzulegen.

10. System nach einem der vorhergehenden Ansprüche, **dadurch gekennzeichnet, dass** der zweite Zweig (8) Volumenstromanpassungsmittel (27) umfasst.

## Claims

1. System for delivering an N₂O/O₂ gas mixture of nitrous oxide (N₂O) and oxygen (O₂) comprising a gas container (1) containing said N₂O/O₂ mixture containing an N₂O content between 40 and 60 mol%, and oxygen for the remainder, fluidically connected to a gas delivery device (2), wherein the gas delivery device (2) comprises:
- an internal gas passage (5) for transporting the gas within the gas delivery device (2), said internal gas passage (5) comprising a gas inlet (6) and divides into a first branch (7) and a second branch (8) each equipped with a gas outlet (10, 11),
- analysis means (3) for measuring an oxygen content of the N₂O/O₂ gas mixture and providing the measured content to control means (4),
- control means (4) configured to compare the measured oxygen content (TOxₘₑₐₛ) provided by the analysis means (3) to a desired oxygen content (TOx_{fix}),
- means (9) for monitoring the supply of gas which are designed to prevent or interrupt the delivery of the gas by the gas delivery device (2) when the control means (4) determine a measured oxygen content (TOxₘₑₐₛ) different from the desired oxygen content (TOx_{fix}),
- a man-machine interface (12) configured to enable one user at least to select a method of delivery of the gas mixture in terms of flow rate or pressure, and
- switching means (13) to supply gas to the first branch (7) or, alternatively, to the second branch (8) of the internal gas passage (5) depending on the method of delivery selected by the user on the man-machine interface (12).

2. System according to the preceding claim, **characterized in that** the gas container (1) contains an N₂O/O₂ gas mixture containing between 45 and 55 mol% of nitrous oxide and oxygen for the remainder.

3. System according to one of the preceding claims, **characterized in that** the gas container (1) contains an equimolar N₂O/O₂ gas mixture.

4. System according to one of the preceding claims, **characterized in that** the analysis means (3) are arranged for measuring the oxygen content of the N₂O/O₂ gas mixture within the internal gas passage (5).

5. System according to one of the preceding claims, **characterized in that** it further comprises alarm means (14) configured to trigger an audible and/or visual alarm when the control means (4) determine a measured oxygen content (TOxₘₑₐₛ) different from the desired oxygen content (TOx_{fix}).

6. System according to one of the preceding claims, **characterized in that** said first branch (7) is a pressure branch and said second branch (8) is a flow rate branch.

7. System according to one of the preceding claims, **characterized in that** the switching means (13) comprise a solenoid valve.

8. System according to one of the preceding claims, **characterized in that** the man-machine interface (12) comprises data entry means (15) and/or means for displaying information (16).

9. System according to one of the preceding claims, **characterized in that** the man-machine interface (12) is configured to enable a user to set a gas administration time and a total care session time.

10. System according to one of the preceding claims, **characterized in that** the second branch (8) comprises flow rate adjustment means (27).
